# EUROPÄISCHE PATENTANMELDUNG

(11) **EP 2 623 135 A1**
(43) Veröffentlichungstag der Anmeldung: **07.08.2013**
(21) Anmeldenummer: 12000659.8
(22) Anmeldetag: 01.02.2012
(51) Int. Cl.: A61L 27/26, A61L 27/56, A61L 27/58

(54) **Matrix zur Zellbesiedelung**

(71) Anmelder: PHRONTIER S.A.R.L., 76490 Caudebec-en-Caux (FR)
(72) Erfinder: Görne, Thomas, DE-22337 Hamburg (DE)
(74) Vertreter: Diehl & Partner GbR

(57) **Zusammenfassung**

Eine Zellimplantat-Matrix weist eine konnektiven Porosität von über 80 % auf und besteht überwiegend aus einem Gemisch bioresorbierbarer Polymere, wobei die Matrix Scheibenform aufweist und wobei eine Oberflächenschicht auf einer Seite der Scheibe weniger als 20 % der durchschnittlichen Porendichte der anderen Seiten aufweist.

Die Matrix wird hergestellt durch Bereitstellen einer bioresorbierbaren Polymerschicht, Aufschichten eines Gemisches aus einem wasserlöslichen Feststoff, mindestens zwei hinsichtlich ihrer Resorptionsrate verschiedenen Polymeren und einem Lösungsmittel für eines der Polymere auf die Polymerschicht; Abdampfen des Lösungsmittels ggf. gefolgt von Kompaktieren des Gemisches; und Wässern des Kompaktkörpers zum Entfernen des Salzes.

## Beschreibung

Die vorliegende Anmeldung bezieht sich auf poröse Matrices zur Zellbesiedlung für therapeutische oder diagnostische Zwecke.

Zellimplantate auf der Basis poröser Matrices aus bioverträglichen Polymeren sind aus WO 2004/108810 A1 bekannt. Bei solchen Matrices sind die Poren vernetzt und dienen als Templat für die Ansiedlung von Zellen *in vivo* (z. B. therapeutisch) oder *in vitro* (z. B. diagnostisch). Bei Transplantationen kann eine solche bioresorbierbare Matrix zur temporären Lokalisation des Transplantats dienen.

Die bekannten Template sind bei einigen Anwendungen noch nicht voll befriedigend, insbesondere hinsichtlich der klinischen Ergebnisse.

Die Erfindung setzt sich daher zum Ziel, eine Verbesserung der klinischen Performance der Template zu erreichen.

Dazu schlägt die Erfindung vor, auf einer Seite des für gewöhnlich scheibenförmigen Templats eine Oberfläche mit weniger als 20 % des durchschnittlichen Porenanteils der anderen Seite(n) bereitzustellen. Diese asymmetrische Struktur ermöglicht, das Templat so im Körper anzuordnen, dass die implantierten vitalen Zellen länger darin bleiben.

Unter einem weiteren Aspekt schlägt die Erfindung Verfahren zur Herstellung von porösen bioresorbierbaren Matrices vor, wobei initial eine Polymerschicht ohne Porenbildner gebildet wird, worauf dann ein Gemisch aus mindestens zwei Polymeren, einem Lösungsmittel für eines der Polymere und einem wasserlöslichen Porenbildner aufgeschichtet wird, gefolgt von Abdampfen des Lösungsmittels und dann Wässern zum Entfernen des Porenbildners. Zwischen diesen letzten beiden Schritten kann in einer Variante noch eine Beaufschlagung mit Druck zum Kompaktieren stattfinden. Beide Verfahren führen zu hochporösen Polymermatrixscheiben, deren eine Seite jedoch eine porenfreie oder zumindest porenarme Membran aufweist. Trotz dieser Membran ist die Versorgung der im Anwendungsfall in den Poren befindlichen Zellen ausreichend, die Verlustraten durch Abwanderung sind jedoch erheblich reduziert. Soll in einer Variante ein solches Templat als Bindegewebsunterstützung dienen, kann die durchgehende Polymerschicht genügend Halt für z. B. Nahtmaterial zur Fixierung des Templats bereitstellen.

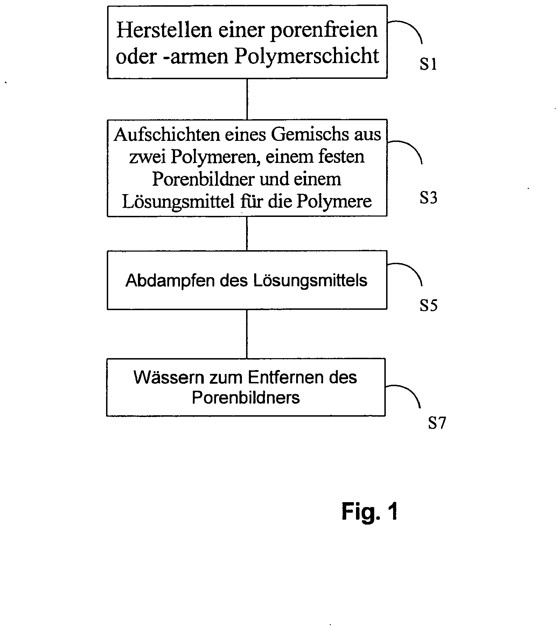

Weitere Merkmale der Erfindung ergeben sich aus der nachfolgenden Beschreibung von Ausführungsbeispielen in Verbindung mit den Ansprüchen sowie den Figuren. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung eines Ausführungsbeispiels wird auf die beiliegenden Figur Bezug genommen, die ein Flussdiagramm für ein erfindungsgemäßes Verfahren zeigt.

In einer Hauptanwendung werden Matrices zur Besiedelung mit Funktionszellen bereitgestellt, beispielsweise mit Hepatozyten und/oder mit Langerhans'schen Inselzellen. Solche biochemischen Funktionszellen heften sich an die Innenwände der Poren der schaumartigen Matrix an (Anheftungsraten über 80 % oder, geeignet beschichtet, über 95 %) und können mit der Matrix in mesotheliale Taschen transplantiert werden, idealerweise des Zellspenders selbst. Dabei wird ausgenutzt, dass in diesem Fall keine Abstoßungsreaktion erfolgt, sondern nur ein vergleichsweise milder, für den therapeutischen Prozess günstiger Fremdkörper-Reiz ausgeübt wird. Binnen weniger Wochen wird die Matrix vaskularisiert und sind die implantierten Zellen nicht mehr nur auf diffusive Versorgung angewiesen. Die Matrices werden so angeordnet, dass die porenarme (oder -freie) Seite innen und die porenreiche Seite außen liegt, um die Verlustrate durch Abwanderung der Zellen niedrig zu halten. Parallel findet eine allmähliche Auflösung eines Teils der abbaubaren Matrix (binnen 3-4 Monaten, oder mindestens 2 und/oder weniger als 7 Monaten) statt und wird das physiologische Milieu dadurch in einer Weise beeinflusst, die ebenfalls dem therapeutischen Erfolg dienlich ist. Es ist zweckmäßig, wenn ein Teil der Polymermischung langsamer erodiert (Verhältnis der Abbauzeiten mindestens 5) und den strukturellen Zusammenhalt längere Zeit, z. B. 2,5-3 Jahre (oder mindestens 2 und/oder weniger als 5 Jahre) gewährleistet. Solche Polymere sind zweckmäßig auf Basis von α-Hydroxycarbonsäuren wie Milchsäure und/oder Glykolsäure, z. B. PLA oder PLGA. Die Hersteller solcher zum Einsatz im menschlichen Körper zugelassener Polymere geben die hier relevanten nominellen Abbauzeiten an.

Zunächst wird eine Lösung eines der verwendeten Polymere in für medizinische Zwecke zugelassenem Chloroform in eine Form gegossen und das Lösungsmittel bei 45°-65° abgedampft. Daraufhin wird eine Polymer-Mischung definierter Partikelgrößen-Verteilung mit einem Kochsalz-Granulat ebenfalls definierter Partikelgrößen-Verteilung gemischt, mit einer Lösung eines der Polymere in Chloroform vermengt und dann auf die bereits hergestellte Polymerschicht gegeben. Aus diesem Vorformling dampft das Lösungsmittel bei leicht erhöhter Temperatur (45°C-65°C) ab und dieser kann dann gewünschtenfalls durch Beaufschlagung mit Druck kompaktiert werden. Anschließend wird der Kompaktkörper gewässert, um durch Entfernen des Salzes die gewünschte Porosität bereitzustellen. Dabei bleibt aber die initial hergestellte Polymerschicht porenfrei. Je nach Einsatzzweck kann die Dicke der porenarmen Schicht durch Menge und Konzentration der anfänglichen Lösung eingestellt werden. Beispielsweise erhält man eine sehr dünne Membran, wenn die Konzentration der Lösung niedrig ist (z. B. 4 %ig in Chloroform, langsam abbaubares Polymer) und die Füllhöhe klein (z. B. 0,1-1 mm, beispielsweise 0,3 mm). Wird eine mechanisch stärker belastbare Struktur gewünscht, kann bei gleicher Konzentration höher (z. B. 5-50 mm, typisch 20-25 mm) eingefüllt werden. Das Abdampfen des Chloroforms dauert dann entsprechend länger (1,5 h). Im ersten Fall hat die entstehende Membran eine Dicke von ca. 10-20 µm, im letzteren Fall von ca. 0,5-1 mm.

Die Kochsalzpartikel der aufzuschichtenden Mischung sind etwas gröber (Median bei 350-370 µm) als die Polymerpartikel (Median des langsamer abbaubaren Polymers zwischen 210 µm und 230 µm, der des schneller abbaubaren Polymers zwischen 150 µm und 170 µm). Dabei sind die Verteilungsbreiten (5 %/95 %) ähnlich, nämlich etwa ± 85-95 µm für Salz bzw. Polymer insgesamt. Die Verteilungsform kann bi-oder trimodal sein. Die Zusammensetzung der Schichtmischung ist zu etwa 96 % Salz, 1-1,5 % festes Polymer und weitere ca. 3-5 % gelöstes Polymer, wobei die Volumenanteile von Feststoffen und Flüssigkeit etwa gleich sind. Insgesamt beträgt der Anteil des schnell abbaubaren Polymers lediglich etwa 5-20 % des Polymers. Die Gesamtdicke der porenbildenden Schicht ist 5-6 mm. In der Variante einer stabileren Initialschicht kann das Salz etwas gröber gewählt werden (Median ca. 400-420 µm). In diesem Fall ist die Gesamtdicke der porenbildenden Schicht 4-5 mm und kann auf das Kompaktieren mit Druck verzichtet werden. Das Wässern dauert ca. 24 h und wird gefolgt von einer Trocknung bei 45-50°C. Wenn eine Beschichtung vorgenommen wird, liegt die Matrix mit der porenarmen Seite auf und wird daher überwiegend die offenporige Seite beschichtet.

Die hier verwendeten Polymere sind z. B. von der Fa. Evonik erhältlich und tragen die Bezeichnungen L210s, L210, L09s, L207s, L206s (langsamer abbaubare PLGA-Polymere) bzw. RG502, RG502H, RG505 (schneller abbaubare PLGA-Polymere).

Bei einer Verwendung außerhalb des Körpers kann eine Matrix gemäß der obigen Beschreibung zur Fixierung von Zellen dienen, die in einem Bioreaktor Agenzien ausgesetzt werden. Beispielsweise können definierte Zelltypen auf diese Weise daraufhin untersucht werden, ob sie auf in Frage kommende Medikamente ansprechen oder nicht, und kann die Therapie in Abhängigkeit von derart erhaltenen Untersuchungsergebnissen geplant werden. Ebenso kann die Medikamentenentwicklung vereinfacht werden, weil Toxizität frühzeitig erkannt wird.

## Patentansprüche

1. Zellimplantat-Matrix mit einer konnektiven Porosität von über 80 %, überwiegend bestehend aus einem Gemisch bioresorbierbarer Polymere, wobei die Matrix Scheibenform aufweist und wobei die Oberfläche auf einer Seite der Scheibe weniger als 20 % des durchschnittlichen Porenanteils der anderen Seiten aufweist.

2. Matrix nach Anspruch 1, wobei die Matrix überwiegend aus Poly(α-hydroxy)carbonsäuren besteht.

3. Matrix nach einem der vorstehenden Ansprüche, wobei sich Resorptionsraten zweier der das Gemisch bildenden Polymere, die jeweils mindestens 10 % des Gemischs ausmachen, um einen Faktor von mehr als 5 unterscheiden.

4. Verwendung der Matrix nach einem der Ansprüche 1 bis 3, zur Besiedelung mit vitalen Zellen und zum Aussetzen der vitalen Zellen einem vorbestimmten Test-Agens außerhalb des Körpers.

5. Herstellung einer Zellimplantat-Matrix, umfassend:
Herstellen einer bioresorbierbaren Polymerschicht;
Aufschichten eines Gemisches aus einem wasserlöslichen Feststoff, mindestens zwei Polymeren mit verschiedenen Resorptionsraten und einem mit Wasser nicht mischbaren Lösungsmittel für wenigstens eines der Polymere auf die Polymerschicht;
Abdampfen des Lösungsmittels; und
Wässern des Agglomerats zum Entfernen des wasserlöslichen Feststoffs.

6. Verfahren nach Anspruch 5, ferner umfassend Beaufschlagen mit Druck zum Kompaktieren des Gemisches nach dem Abdampfen des Lösungsmittels.

7. Verfahren nach Anspruch 5 oder 6, wobei als Lösungsmittel Chloroform verwendet wird.

8. Verfahren nach einem der Ansprüche 5 bis 7, wobei sich Resorptionsraten zweier der das Gemisch bildenden Polymere, die jeweils mindestens 10 % des Gemischs ausmachen, um einen Faktor von mehr als 5 unterscheiden.

9. Verfahren nach einem der Ansprüche 5 bis 8, wobei die Dicke der initial hergestellten Schicht 0,002-2,5 mm beträgt.

10. Verfahren nach Anspruch 9, wobei die Dicke der initial hergestellten Schicht 0,005-0,025 mm beträgt.

11. Verfahren nach Anspruch 9, wobei die Dicke der initial hergestellten Schicht 0,25-2,0 mm beträgt.

12. Verwendung der nach dem Verfahren gemäß einem der Ansprüche 5 bis 11 hergestellten Matrix, zur Besiedelung mit vitalen Zellen und zum Aussetzen der vitalen Zellen einem vorbestimmten Test-Agens außerhalb des Körpers.
